# FASCICULE DE BREVET EUROPEEN

(11) **EP 4 606 408 B1**
(45) Date de publication et mention de la délivrance du brevet: **29.07.2026**
(21) Numéro de dépôt: 25156378.9
(22) Date de dépôt: 06.02.2025
(51) Int. Cl.: A61M 16/00, A61M 16/12, A61M 16/20

(54) **INSTALLATION DE FOURNITURE DE NO À UN PATIENT CONFIGURÉE POUR FOURNIR UN DÉBIT DE SECOURS EN CAS DE DYSFONCTIONNEMENT DU CAPTEUR DE DÉBIT**
INSTALLATION ZUR NO-VERSORGUNG EINES PATIENTEN MIT KONFIGURATION ZUR BEREITSTELLUNG EINES NOTFLUSSES IM FALL EINER FEHLFUNKTION DES STRÖMUNGSSENSORS
INSTALLATION FOR SUPPLYING NO TO A PATIENT CONFIGURED TO PROVIDE AN EMERGENCY FLOW IN CASE OF A MALFUNCTION OF THE FLOW SENSOR

(30) Priorité: 23.02.2024 FR 2401784
(43) Date de publication de la demande: 27.08.2025
(73) Titulaire: INOSYSTEMS, 92160 Antony (FR)
(72) Inventeur: MARCHAL, Frederic, 92160 Antony (FR); SCHMITT, Mary, 92160 Antony (FR); BOULANGER, Thierry, 92160 Antony (FR); BLANDIN, Yann, 92160 Antony (FR)
(74) Mandataire: Air Liquide

(56) Documents cités:
- EP-A1- 4 209 243
- EP-B1- 3 233 171
- EP-B1- 3 410 927
- FR-A1- 3 133 318
- US-A1- 2023 226 291

## Description

L'invention concerne une installation de fourniture d'un mélange gazeux à base de NO à un patient, typiquement un mélange NO/azote (N₂), comprenant un appareil de délivrance de NO apte à délivrer un débit de NO de secours en cas de dysfonctionnement se traduisant par une interruption de transmission par le capteur de débit aux moyens de pilotage de l'appareil de délivrance de NO, de toute mesure de débit de gaz respiratoire à base d'oxygène, tel de l'air ou un mélange NO/N₂, provenant d'un ventilateur médical, typiquement d'un gaz respiratoire provenant d'un ventilateur médical, c'est-à-dire en cas de perte du signal de débit.

Le monoxyde d'azote inhalé (NO ou NOi) est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Pour mettre en œuvre une thérapie par NO inhalé, on utilise une installation de fourniture de gaz, aussi appelée installation d'administration de NO, comprenant un appareil de délivrance de NO et un ventilateur médical, c'est-à-dire un appareil d'assistance respiratoire, alimentant un circuit patient. L'appareil de délivrance de NO permet d'injecter un mélange gazeux à base de NO, typiquement un mélange NO/azote, dans le circuit patient alimenté par ailleurs en un flux gazeux contenant de l'oxygène (au moins environ 20% vol.), tel de l'air ou un mélange oxygène/azote (O₂/N₂), fourni par le ventilateur médical. Le circuit patient comprend en général un ou plusieurs conduits flexibles reliés fluidiquement à une interface respiratoire, telle une sonde d'intubation trachéale ou analogue, servant à délivrer au patient à traiter, un mélange gazeux thérapeutique contenant une quantité ou dose donnée de NO, c'est-à-dire une posologie, typiquement entre 5 et 40 ppmv de NO.

Une telle installation de fourniture de gaz est décrite par exemple par EP3821929. Ce type d'installation est utilisé en milieu hospitalier pour administrer le traitement par NO et ainsi soigner les patients ayant besoin d'inhaler du NO pour traiter leur hypertension artérielle pulmonaire.

Des installations de ce type sont également décrites par EP4209243, EP4241817, EP4241812 et EP4295882.

Afin de pouvoir fournir un mélange gazeux thérapeutique aux patients contenant une teneur en NO correspondant à la posologie désirée, l'appareil de délivrance de NO doit comprendre des moyens ou un système de contrôle de débit permettant de contrôler ou ajuster le débit de NO/N₂ fourni, par exemple un système incluant une ou des vannes proportionnelles ou analogues contrôlées par des moyens de pilotage de l'appareil.

Or, le débit de NO/N₂ à fournir dépend notamment du débit de gaz respiratoire (i.e. air ou O₂/N₂) provenant du ventilateur médical.

Dès lors, on utilise habituellement un capteur de débit agencé dans le circuit respiratoire entre le ventilateur médical et le site d'injection du mélange NO/N₂, pour opérer, en (quasi-)continu des mesures de débit de gaz respiratoire. Ces mesures de débit sont ensuite transmises aux moyens de pilotage qui les utilisent pour calculer un débit de NO de consigne qui sert à contrôler les moyens de contrôle de débit, typiquement une ou des vannes proportionnelles, ou autres.

Or, pendant l'utilisation d'une installation de fourniture de NO, il arrive que la mesure de débit de gaz respiratoire par le capteur de débit soit perturbée, défectueuse ou impossible à réaliser, par exemple en cas de dysfonctionnement ou de débranchement accidentel du capteur de débit, engendrant alors une interruption dans la transmission des mesures de débit par le capteur de débit.

Cette interruption de transmission des mesures de débit pose un problème majeur de sécurité pour le patient car en l'absence de mesure de débit, les moyens de pilotage ne peuvent plus calculer le débit de NO de consigne qui sert à contrôler les moyens de contrôle de débit, donc la délivrance de NO se fait de manière erronée ou peut même s'interrompre.

Pour éviter cela, l'appareil doit pouvoir fournir le NO au patient à la posologie souhaitée, en passant dans un mode de fourniture dit de « secours » ou de « dosage d'urgence », dans lequel le débit de NO de consigne est calculé même en l'absence de mesure de débit, c'est-à-dire même en cas d'interruption de la transmission des mesures de débit par le capteur de débit aux moyens de pilotage.

Ainsi, EP3233171 propose de fournir un débit fixé prédéterminé de mélange NO/N₂ en cas de détection d'une interruption de transmission des mesures de débit par le capteur de débit. Ceci n'est pas précis et conduit à des fluctuations importantes de la teneur en NO du mélange gazeux combiné puisque le débit de NO/N₂ est fixe alors que celui du flux de gaz respiratoire, dans lequel est injecté le débit de NO/N₂ pour former le mélange gazeux combiné, varie au fil du temps.

Par ailleurs, EP3410927 propose de mémoriser un historique de valeurs de mesures de débit opérées par le capteur de débit et d'utiliser cet historique de valeurs mémorisées pour calculer le débit de NO/N₂ de consigne, en cas d'interruption de transmission des mesures de débit. Cette solution n'est pas idéale car les valeurs de débit « brutes » mémorisées peuvent être erronées, notamment en cas de dysfonctionnement intermittent du capteur de débit pouvant par exemple résulter de décharges électrostatiques intempestives sur le capteur, conduisant à mémoriser des valeurs de débit de gaz respiratoire incorrectes. Les valeurs de débits étant erronées, les calculs de débit les utilisant sont dès lors aussi erronés, ce qui engendre une fourniture d'une quantité de NO/N₂ inadéquate.

En outre, EP3410927 propose d'utiliser un capteur de NO additionnel pour mesurer le débit de NO/N₂ fourni par l'appareil au fil du temps et de mémoriser un historique des mesures de débit de NO opérées par le capteur de NO additionnel. En cas d'interruption de transmission des mesures de débit de gaz respiratoire par le capteur de gaz respiratoire, l'appareil utilise l'historique de valeurs de débit de NO mémorisées pour fixer le débit de NO/N₂ de consigne. Là encore, la solution n'est pas idéale car elle complique l'architecture globale de l'appareil en nécessitant l'incorporation d'un capteur additionnel. De plus, travailler sur les valeurs historiques de NO précédemment délivré présente des inconvénients étant donné que ces valeurs de débit peuvent être erronées, du fait par exemple d'un ratio signal/bruit de mesure trop important, en particulier pour les valeurs de NO délivrées qui sont très faibles (i.e. ppmv).

Un problème est donc de proposer une installation de délivrance de NO améliorée, permettant de déterminer un débit de NO de consigne, en l'absence de mesure de débit de gaz respiratoire, c'est-à-dire en cas d'interruption de la transmission aux moyens de pilotage (i.e. perte du signal de débit respiratoire), des mesures de débit de gaz respiratoire opérées par le capteur de débit, de sorte de pouvoir fournir un mélange gazeux final à base de NO au patient contenant une proportion de NO égale ou proche d'une posologie fixée par un personnel soignant, i.e. un médecin ou analogue.

Une solution de l'invention concerne une installation de fourniture d'un mélange gazeux contenant du NO, aussi appelée « installation de fourniture de NO », à un patient comprenant :
- un appareil de délivrance de NO alimenté en un gaz contenant du NO en une proportion initiale donnée, typiquement un mélange NO/N₂, et configuré pour fournir le gaz contenant du NO,
- un circuit respiratoire comprenant un dispositif d'injection configuré pour opérer un mélange du gaz contenant du NO provenant de l'appareil de délivrance de NO avec un flux de gaz respiratoire contenant de l'O₂ acheminé par le circuit respiratoire, et obtenir un mélange gazeux combiné contenant du NO et de l'oxygène, et
- un capteur de débit configuré pour mesurer au moins un débit de gaz respiratoire au sein du circuit respiratoire et fournir au moins une mesure de débit de gaz respiratoire.

L'appareil de délivrance de NO de l'installation de fourniture de NO comprend :
- un circuit de gaz interne pour acheminer le gaz contenant du NO,
- des moyens de contrôle de débit principaux et secondaires configurés pour contrôler (i.e. autoriser, empêcher/interdire, ajuster...) le débit de gaz contenant du NO acheminé par le circuit de gaz interne,
- des moyens de pilotage à (micro)processeur configurés pour :
   ▪ déterminer au moins un débit de consigne de gaz contenant du NO à fournir au dispositif d'injection, à partir d'au moins une mesure de débit de gaz respiratoire opérée par le capteur de débit, et
   ▪ piloter au moins une partie des moyens de contrôle de débit principaux et secondaires pour fournir le gaz contenant du NO au débit de consigne ayant été déterminé,
- et des moyens de mémorisation.

De plus, dans l'installation de fourniture de NO (i.e. monoxyde d'azote), tel un mélange NO/N₂ :
- les moyens de mémorisation sont configurés pour mémoriser des débits de consigne successifs de gaz contenant du NO ayant été déterminés par les moyens de pilotage, et
- en cas d'interruption de transmission aux moyens de pilotage, de toute mesure de débit de gaz respiratoire par le capteur de débit (i.e. en cas de perte du signal de débit respiratoire), les moyens de pilotage sont configurés pour piloter les moyens de contrôle de débit principaux et/ou secondaires pour fournir le gaz contenant du NO à un débit de secours obtenu ou calculé à partir d'un ou plusieurs débits de consigne de gaz contenant du NO ayant été déterminés par les moyens de pilotage et mémorisés par les moyens de mémorisation, avant ladite interruption de transmission.

Selon le mode de réalisation considéré, l'installation de l'invention peut comprendre l'une ou plusieurs des caractéristiques suivantes :
- les moyens de pilotage sont configurés pour déterminer des débits de consigne de gaz contenant du NO successifs à partir de plusieurs mesures de débit de gaz respiratoire successives opérées par le capteur de débit et fournies auxdits moyens de pilotage.
- les moyens de mémorisation sont configurés pour mémoriser les débits de consigne de gaz contenant du NO successifs ayant été déterminés par les moyens de pilotage.
- les moyens de mémorisation sont configurés pour mémoriser les débits de consigne de gaz contenant du NO successifs ayant été déterminés pendant le fonctionnement normal de l'appareil et/ou de l'installation, c'est-à-dire avant toute perte du signal de débit respiratoire.
- le débit de secours est obtenu ou calculé à partir de plusieurs débits de consigne ayant été déterminés par les moyens de pilotage pendant une durée (dt) donnée, avant ladite interruption de transmission, c'est-à-dire des débits successifs déterminées pendant le fonctionnement normal de l'appareil.
- le débit de secours est calculé en opérant une moyenne des débits de consigne ayant été mémorisés pendant la durée donnée (dt), c'est-à-dire avant toute perte du signal de débit respiratoire.
- la durée (dt) donnée est inférieure ou égale à 30 secondes, de préférence inférieure ou égale à 20 secondes, de préférence encore inférieure ou égale à 10 secondes.
- la durée (dt) donnée est fixe ou, selon un autre mode de réalisation, modifiable.
- la durée (dt) donnée est mémorisée.
- les moyens de pilotage sont configurés pour déterminer ledit au moins un débit de consigne de gaz contenant du NO à fournir au dispositif d'injection, à partir d'au moins une mesure de débit de gaz respiratoire opérée et fournie par le capteur de débit, d'une consigne de teneur en NO correspondant à la proportion finale de NO souhaitée (i.e. une posologie) dans le mélange gazeux combiné, et de la proportion initiale de NO dans le gaz contenant du NO alimentant l'appareil de délivrance de NO.
- la proportion initiale de NO dans le gaz contenant du NO alimentant l'appareil de délivrance de NO est mémorisée au sein des moyens de mémorisation.
- en cas d'interruption de transmission aux moyens de pilotage, de toute mesure de débit de gaz respiratoire par le capteur de débit (i.e. en cas de perte du signal de débit respiratoire), les moyens de pilotage sont configurés pour piloter les moyens de contrôle de débit principaux (i.e. qui restent opérationnels, c'est-à-dire aux moyens de contrôle de débit principaux qui ne dysfonctionnent pas) pour fournir le gaz contenant du NO audit débit de secours.
- lorsque les moyens de contrôle de débit principaux continuent à fonctionner normalement (i.e. qui ne dysfonctionnent pas), malgré l'interruption de transmission des mesures de débit (i.e. en cas de perte du signal de débit respiratoire), le gaz contenant le NO transite par la ligne principale comprenant les moyens de contrôle de débit principaux qui sont pilotées par les moyens de pilotage afin de fournir le gaz contenant du NO audit débit de secours.
- à l'inverse, en cas d'interruption de transmission aux moyens de pilotage de toute mesure de débit de gaz respiratoire par le capteur de débit et par ailleurs de dysfonctionnement des moyens de contrôle de débit principaux (donc de moyens de contrôle de débit principaux non-opérationnels ou hors service), les moyens de pilotage sont configurés pour piloter les moyens de contrôle de débit secondaires (pour les rendre opérationnels) afin de fournir le gaz contenant du NO audit débit de secours.
- lorsque les moyens de contrôle de débit principaux ne fonctionnent pas normalement, i.e. sont dysfonctionnels, avec par ailleurs interruption de transmission des mesures de débit (i.e. en cas de perte du signal de débit respiratoire), le gaz contenant le NO transite par la ligne secondaire, (et non plus par la ligne principale) comprenant les moyens de contrôle de débit secondaires qui sont pilotées par les moyens de pilotage afin de fournir le gaz contenant du NO audit débit de secours.
- le capteur de débit est agencé au sein du circuit respiratoire, en amont du dispositif d'injection.
- l'appareil de délivrance de NO est alimenté en un gaz contenant une proportion initiale de NO comprise entre 100 et 1500 ppmv, typiquement entre 200 et 1000 ppmv.
- l'appareil de délivrance de NO est alimenté en un mélange gazeux formé d'azote et de NO.
- l'appareil de délivrance de NO comprend des moyens de réglage de dose configurés pour permettre à un utilisateur de fixer ou sélectionner la consigne de teneur en NO correspondant à la proportion finale de NO souhaitée dans le mélange gazeux combiné, i.e. une posologie.
- les moyens de réglage de dose font partie d'une IHM (interface homme-machine) ou IGU (interface graphique utilisateur).
- les moyens de réglage de dose comprennent une ou des touches tactiles, actionnables par l'utilisateur, s'affichant sur un écran digital à dalle tactile de l'IHM
- l'écran de l'IHM est du type à affichage en couleurs.
- la consigne de teneur en NO est comprise entre 1 et 80 ppmv, typiquement entre 5 et 40 ppmv.
- elle comprend un ventilateur médical configuré pour fournir le flux de gaz respiratoire contenant de l'O₂ audit circuit respiratoire.
- le ventilateur médical est configuré pour fournir un flux de gaz respiratoire contenant au moins 20%vol. environ d'O₂, typiquement un mélange NO/N₂ ou de l'air.
- l'appareil de délivrance de NO et le ventilateur médical sont raccordés fluidiquement au circuit respiratoire.
- le capteur de débit est agencé dans le circuit respiratoire entre le ventilateur médical et le dispositif d'injection.
- le dispositif d'injection comprend une première entrée de gaz alimentée en flux de gaz respiratoire contenant de l'O₂, i.e. provenant du ventilateur médical.
- le dispositif d'injection comprend en outre une seconde entrée de gaz alimentée en gaz contenant du NO audit débit de consigne, i.e. provenant de l'appareil de délivrance de NO.
- le dispositif d'injection comprend en outre une sortie de gaz fournissant le mélange gazeux combiné contenant du NO et de l'oxygène, obtenu par mélange, au sein du dispositif d'injection, du gaz contenant du NO (e.g. mélange NO/N₂) avec le flux de gaz respiratoire contenant de l'O₂ (e.g. air ou mélange O₂/N₂).
- les moyens de mémorisation comprennent une mémoire informatique, telle une mémoire flash, une RAM ou analogue.
- les moyens de pilotage sont configurés pour détecter toute interruption de transmission des mesures de débit de gaz respiratoire par le capteur de débit aux moyens de pilotage.
- les moyens de pilotage sont configurés pour détecter toute interruption de transmission des mesures de débit de gaz respiratoire opérées par le capteur de débit, c'est-à-dire toute perte du signal de débit respiratoire.
- l'appareil de délivrance de NO fonctionne selon au moins deux modes de fonctionnement comprenant un mode de fonctionnement normal et un mode de fonctionnement de secours, i.e. un mode de secours .
- l'appareil de délivrance de NO est configuré pour passer automatiquement du mode de fonctionnement normal au mode de secours en réponse à une interruption de transmission des mesures de débit de gaz respiratoire opérées par le capteur de débit (i.e. en cas de perte du signal de débit respiratoire) et ce, avec ou sans dysfonctionnement des moyens de contrôle de débit principaux, en particulier des moyens à vanne principaux.
- une détection par les moyens de pilotage sont configurés pour détecter toute interruption de transmission des mesures de débit de gaz respiratoire et/ou tout dysfonctionnement des moyens de contrôle de débit principaux
- les moyens de pilotage sont configurés pour activer le mode de secours en cas d'interruption de transmission des mesures de débit de gaz respiratoire (i.e. en cas de perte du signal de débit respiratoire).
- les moyens de pilotage sont configurés pour déterminer des débits de consigne de gaz contenant du NO successifs à partir de mesures de débit de gaz respiratoire successives opérées par le capteur de débit pendant un fonctionnement normal de l'appareil de délivrance de NO, c'est-à-dire lorsqu'il opère selon le mode de fonctionnement normal.
- les moyens de pilotage sont configurés pour commander les moyens de contrôle de débit principaux et/ou secondaires pour fournir le gaz contenant du NO au débit de secours, pendant un fonctionnement de l'appareil de délivrance de NO en mode de secours.
- les moyens de pilotage sont configurés pour déterminer et mémoriser les débits de consigne de gaz contenant du NO successifs pendant le fonctionnement normal de l'appareil de délivrance de NO.
- le circuit de gaz interne comprend deux tronçons de gaz agencés en parallèle comprenant un tronçon principal et un tronçon secondaire ou tronçon de secours.
- les tronçons principal et secondaire comprennent des moyens de contrôle de débit principaux et secondaires, typiquement des moyens à vannes principaux et secondaires, telles des électrovannes ou analogues.
- les moyens de pilotage sont configurés pour contrôler les moyens de contrôle de débit principaux et secondaires pour autoriser ou empêcher le passage du flux de NO/N₂ dans l'un ou l'autre des tronçons principal et secondaire, en particulier en fonction du mode de fonctionnement de l'appareil de délivrance de NO.
- en mode de fonctionnement normal, les moyens de pilotage sont configurés pour contrôler les moyens de contrôle de débit principaux et/ou secondaires pour que le flux de NO/N₂ transite uniquement par le tronçon principal.
- en mode secours, les moyens de pilotage sont configurés pour contrôler les moyens de contrôle de débit principaux et/ou secondaires pour que le flux de NO/N₂ transite par le tronçon principal ou, selon le cas, par le tronçon de secours selon que les moyens de contrôle de débit principaux fonctionnent ou, au contraire, sont dysfonctionnels, respectivement.
- les moyens de contrôle de débit principaux et secondaires comprennent des moyens à vannes principaux et secondaires, telles des électrovannes.
- les moyens de contrôle de débit principaux comprennent une électrovanne proportionnelle.
- les moyens de contrôle de débit secondaires comprennent une électrovanne tout ou rien, de préférence pilotée en mode pulsé.
- les moyens de contrôle de débit principaux comprennent un contrôleur de débit massique (Mass Flow Controler).
- le contrôleur de débit massique (Mass Flow Controler) ou MFC comprend au moins une électrovanne proportionnelle pilotée par les moyens de pilotage et un capteur de débit principal.
- le capteur de débit principal est intégré à la structure de l'électrovanne proportionnelle du MFC.
- l'électrovanne du MFC embarque en outre une carte électronique à microprocesseur mettant en œuvre au moins un algorithme.
- le tronçon secondaire, i.e. le tronçon de secours, comprend un dispositif à orifice calibré servant à contrôler le débit de gaz circulant dans le tronçon secondaire, en particulier en mode secours.
- le dispositif à orifice calibré est agencé en aval de moyens de contrôle de débit secondaires du tronçon secondaire.
- les moyens de contrôle de débit secondaires du tronçon secondaire comprennent des moyens à vannes secondaires, typiquement une électrovanne, de préférence de type tout ou rien (TOR).
- l'électrovanne de type tout ou rien (TOR) est pilotée par les moyens de pilotage, de préférence en mode pulsé.
- le tronçon principal comprend une (ou des) électrovanne proportionnelle et le tronçon secondaire comprend une (ou des) électrovanne de type tout ou rien (TOR).
- le tronçon principal comprend une (ou des) électrovanne proportionnelle qui est normalement fermée.
- en fonctionnement normal, l'électrovanne proportionnelle est pilotée pour être au moins partiellement ouverte (i.e. ouverture proportionnelle) et laisser passer du gaz contenant du NO.
   en fonctionnement normal, l'électrovanne TOR est fermée ou pilotée par les moyens de pilotage pour être fermée, et interdire tout passage de gaz contenant du NO, i.e. empêcher la circulation de gaz.
- en cas de dysfonctionnement des moyens de contrôle de débit principaux, typiquement du MFC, l'électrovanne proportionnelle se ferme automatiquement ou selon le cas, est pilotée pour se fermer, de manière à interdire, stopper ou empêcher toute circulation de gaz.
- en cas de dysfonctionnement des moyens de contrôle de débit principaux, typiquement du MFC, l'électrovanne TOR s'ouvre automatiquement ou est pilotée pour être ouverte (i.e. pour s'ouvrir), de manière à autoriser ou permettre un passage de gaz (e.g. NO/N₂) dans le tronçon secondaire.
- les moyens de pilotage comprennent un (micro)contrôleur ou analogue.
- les moyens de pilotage comprennent un (ou des) (micro)processeur agencé sur une (ou des) carte électronique.
- les moyens de pilotage comprennent un (ou des) (micro)processeur mettant en œuvre un ou des algorithmes, notamment un (ou des) algorithme de pilotage des moyens de contrôle de débit principaux et/ou secondaires, un (ou des) algorithme de traitement des mesures de débit de gaz respiratoire....
- les moyens de mémorisation sont intégrés aux moyens de pilotage, en particulier agencés sur la carte électronique.

De plus, selon le mode de réalisation considéré, l'installation de fourniture de gaz de l'invention peut comprendre l'une ou plusieurs des caractéristiques additionnelle suivantes :
- le ventilateur médical délivre de l'air ou un mélange oxygène/azote, i.e. en tant que gaz respiratoire contenant au moins 20% vol. environ d'oxygène, de préférence au moins 21% vol. environ d'oxygène.
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- la source de NO contient un mélange gazeux NO/N₂ contenant entre 100 et 2000 ppmv de NO, le reste étant de l'azote (N₂), de préférence entre 100 et 1000 ppmv de NO, conditionné à une pression comprise entre 10 et 250 bar abs, typiquement à plus de 100 bar abs (avant début de soutirage).
- la source de NO est ou comprend une (ou des) bouteille de gaz ayant une contenance comprise entre 0,5 et 50 L (équivalent en eau).
- la (les) bouteille de gaz comprend un corps cylindrique en acier ou en alliage d'aluminium et est équipée d'un robinet simple (sans détendeur) ou à détendeur intégré ou RDI, de préférence un RDI, protégé par un capotage de protection, par exemple en métal ou polymère.
- le circuit respiratoire de l'installation comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire, ou autre.
- la branche inspiratoire et la branche expiratoire sont en outre fluidiquement raccordées à, respectivement, des orifices de sortie et d'entrée du ventilateur médical.
- le circuit respiratoire, en particulier la branche inspiratoire, peut comprendre un humidificateur de gaz.
- l'humidificateur de gaz est agencé en aval du dispositif d'injection, par exemple un module d'injection de NO, de manière à pouvoir humidifier le gaz avant son administration par inhalation au patient.
- le ventilateur et l'appareil de délivrance de NO sont alimentés électriquement par une ou des sources de courant électrique, typiquement le secteur (110/220V) et/ou une ou des batteries rechargeables.

Selon un autre aspect (non revendiqué), la présente divulgation concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO assurant une délivrance de NO à un débit de secours compatible avec la posologie souhaitée et ce, même en cas de dysfonctionnement empêchant toute échange de mesures entre le capteur de débit mesurant le débit du gaz respiratoire à base d'oxygène fourni par le ventilateur médical (e.g. air ou N₂/O₂) et les moyens de pilotage de l'appareil de délivrance de NO, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les termes « concentration », « quantité », « proportion », « dose » et « teneur » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « fonctionnement normal » ou « mode de fonctionnement normal », on entend un fonctionnement habituel de l'appareil de délivrance de NO en l'absence de tout dysfonctionnement entravant ou empêchant une fourniture (i.e. interruption de transmission ou perte de signal de débit) aux moyens de pilotage de l'appareil de délivrance de NO, des mesures de débit (i.e. valeurs ou signaux) par le capteur de débit mesurant le débit du gaz respiratoire (i.e. air ou N₂/O₂) acheminé par le circuit respiratoire, typiquement provenant du ventilateur médical.
- par « dysfonctionnement », on entend une panne, une anomalie, un problème, un défaut, un débranchement accidentel ou tout problème technique, comme par exemple des perturbations électromagnétiques, affectant le fonctionnement normal de l'appareil de délivrance de NO en empêchant la fourniture aux moyens de pilotage et/ou la réception par lesdits moyens de pilotage (i.e. interruption de transmission du signal ou perte de signal de débit), des mesures de débit opérées par le capteur de débit mesurant le débit du gaz respiratoire (i.e. air ou N₂/O₂) acheminé par le circuit respiratoire, typiquement provenant du ventilateur médical.
- par « mesure de débit », on entend une valeur de débit (e.g. une valeur numérique) ou un signal représentatif d'une telle valeur de débit reflétant ou correspondant à un débit gazeux mesuré par un capteur de débit, tel un capteur de débit massique.
- par « mesure de pression », on entend une valeur de pression (e.g. une valeur numérique) ou un signal représentatif d'une telle valeur de pression reflétant ou correspondant à la pression gazeuse mesurée par un capteur de pression (quel que soit le mode de fonctionnement de ce capteur).
- par « fonctionnement en mode secours », on entend un fonctionnement de l'appareil de délivrance de NO en cas de dysfonctionnement entravant ou empêchant la fourniture (i.e. interruption de transmission du signal de débit ou perte du signal de débit) aux moyens de pilotage de l'appareil de délivrance de NO, des mesures de débit du flux de gaz respiratoire (i.e. air ou mélange O₂/N₂) opérées par le capteur de débit, avec ou sans dysfonctionnement éventuel concomitant des moyens de contrôle de débit principaux (e.g. de l'électrovalve proportionnelle du MFC) situés sur le tronçon principal de gaz.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'administration de gaz selon l'invention.
Fig. 2 schématise un mode de réalisation de l'architecture interne de l'appareil de délivrance de NO d'une installation d'administration de gaz selon l'invention, en particulier une installation d'administration de gaz selon Fig. 1.

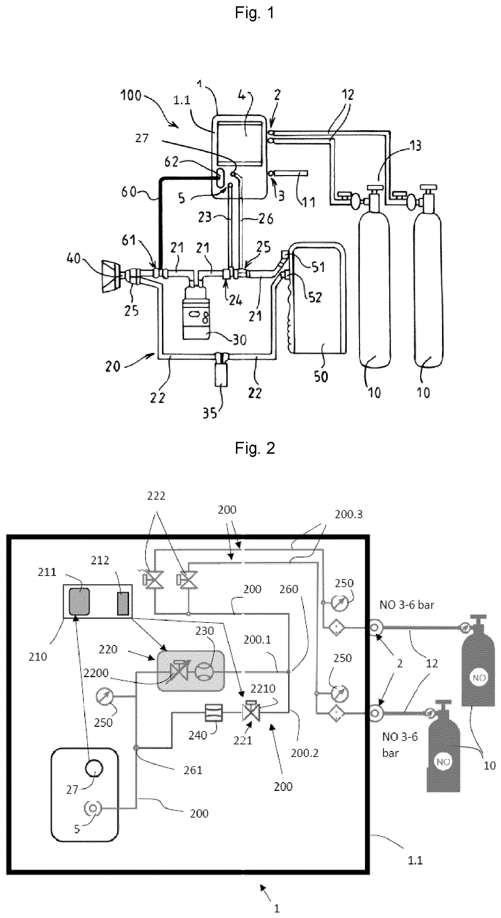

Fig. 1 schématise un mode de réalisation d'une installation d'administration de gaz 100 selon l'invention comprenant un appareil de fourniture de NO 1 fournissant un mélange gazeux à base de monoxyde d'azote (NO), et un ventilateur médical 50 fournissant un gaz contenant au moins 20%vol. d'oxygène, tel de l'air ou autre.

L'installation 100 comprend ici deux bouteilles de gaz sous pression 10 contenant chacune un mélange gazeux NO/N₂, à savoir ici un mélange gazeux NO/N₂ contenant entre 100 et 1000 ppmv de NO (reste N₂), par exemple 450 ou 800 ppm vol. de NO (reste N₂), ou toute autre concentration adéquate, qui alimentent en mélange NO/N₂, le dispositif ou appareil 1 de délivrance ou fourniture de NO permettant de suivre et contrôler la fourniture du mélange gazeux NO/N₂.

Les bouteilles de gaz 10 sont reliées fluidiquement à l'appareil 1 de fourniture de NO, via des lignes d'amenée de gaz 12, tel que des tuyaux ou conduits flexibles ou analogues, qui peuvent être équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 de l'appareil de délivrance de NO 1 qui alimentent un circuit de gaz interne 200, comme schématisé en Fig. 2, servant à acheminer le gaz au sein de l'appareil de fourniture de NO 1, c'est-à-dire dans le boitier ou carcasse 1.1 externe de l'appareil 1.

Dans le mode de réalisation de Fig. 2, le circuit de gaz interne 200 est relié à deux entrées de gaz 2 agencées en parallèle et alimentant chacune un tronçon d'entrée 200.3 dédié du circuit de gaz interne 200. Des vannes de contrôle 222 ou analogue contrôlent le passage du flux de NO/N₂ dans ces tronçon d'entrée 200.3.

L'appareil de délivrance de NO 1 comprend par ailleurs une entrée d'oxygène 3 reliée fluidiquement, via une ligne d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier. Ceci permet d'alimenter le circuit de gaz interne 200 en oxygène quand cela est nécessaire.

Le ventilateur médical 50, c'est-à-dire un appareil d'assistance respiratoire, fournit un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂).

Le ventilateur médical 50 et l'appareil de fourniture de NO 1 de l'installation 100 sont en communication fluidique avec un circuit respiratoire 20, aussi appelé circuit patient, en particulier avec une ligne d'alimentation en gaz ou branche inspiratoire 21 du circuit respiratoire 20, qui sert à acheminer le flux gazeux vers l'interface respiratoire 40 fournissant le flux gazeux thérapeutique au patient, c'est-à-dire un mélange gazeux final contenant la posologie en NO souhaitée.

Plus précisément, le mélange gazeux final à administrer au patient est formé par mélange du flux à base d'oxygène (e.g. air ou mélange NO/N₂) provenant du ventilateur médical 50 et du flux contenant le NO, i.e. le mélange gazeux NO/N₂, délivré par l'appareil de délivrance de NO 1.

Pour ce faire, l'appareil de délivrance de NO 1 fournit ou injecte le mélange NO/N₂ dans le circuit respiratoire 20, typiquement dans la branche inspiratoire 21, via un conduit ou une ligne d'injection 23, reliant fluidiquement le circuit de gaz interne de l'appareil de fourniture de NO 1 à un dispositif d'injection 24 agencé sur la ligne d'alimentation en gaz 21.

Le dispositif d'injection 24 est configuré pour opérer un mélange du gaz contenant du NO provenant de l'appareil de délivrance de NO 1 avec le flux de gaz respiratoire contenant de l'O₂ provenant du ventilateur 50 et acheminé par la branche inspiratoire 21 du circuit respiratoire 20, et obtenir un mélange gazeux combiné contenant du NO et de l'oxygène, c'est-à-dire le mélange gazeux final administré au patient.

Plus précisément, le dispositif d'injection 24 comprend une première entrée de gaz alimentée en flux de gaz respiratoire contenant de l'O₂ provenant du ventilateur médical 50, une seconde entrée de gaz alimentée en gaz contenant du NO, i.e. provenant de l'appareil de délivrance de NO 1, et une sortie de gaz fournissant le mélange gazeux combiné contenant du NO et de l'oxygène, obtenu par mélange, au sein du dispositif d'injection 24, du gaz contenant du NO avec le flux de gaz respiratoire contenant de l'O₂.

Autrement dit, le flux de NO/N₂ amené par la ligne d'injection 23 se mélange alors (grâce au dispositif d'injection 24) au flux de gaz à base d'oxygène (> 20% d'O₂), e.g. de l'air ou un mélange oxygène/azote, délivré par le ventilateur médical 50 et véhiculé par la branche inspiratoire 21 du circuit patient 20 de sorte d'obtenir un mélange final, i.e. un mélange combiné, à administrer au patient contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux final NO/N₂/O₂.

La branche inspiratoire 21 du circuit 20 comprend en outre un humidificateur de gaz 30 agencé en aval du dispositif d'injection 24. Il permet d'humidifier le flux de gaz final, e.g. le mélange gazeux combiné NO/N₂/O₂, avant qu'il ne soit administré par inhalation au patient à traiter, au moyen d'une interface respiratoire 40, telle une sonde d'intubation trachéale, un masque respiratoire ou analogue.

Une ligne de récupération des gaz expirés par le patient forme une branche expiratoire 22 du circuit patient 20. Elle est reliée fluidiquement à la branche inspiratoire 21 via une pièce de raccordement 25, telle une pièce en Y.

La branche inspiratoire 21 est, à son extrémité amont, raccordée fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂ fourni par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient. La branche expiratoire 22 peut comprendre un ou plusieurs composants optionnels, par exemple un dispositif d'élimination du CO₂ 35, i.e. un piège à CO₂, tel un bac à chaud ou autre, permettant d'éliminer le CO₂ présent dans les gaz expirés par le patient, un filtre ou autre.

Par ailleurs, un capteur de débit 25, par exemple du type à fil chaud ou à différentiel de pression, est agencé sur le circuit respiratoire 20, en particulier sur la branche inspiratoire 21, entre le ventilateur 50 et le dispositif d'injection 24. Le capteur de débit 25 est relié à un port de connexion au capteur 27, de l'appareil de délivrance de NO 1, via une (des) ligne de mesure de débit 26 venant se raccorder audit port de connexion au capteur 27. Il sert à mesurer le débit de gaz délivré par le ventilateur 50, tel de l'air ou N₂/O₂, circulant dans la branche inspiratoire 21, en amont du dispositif d'injection.

Ces mesures de débit opérées par le capteur de débit 25 permettent de contrôler ou réguler plus efficacement la délivrance du flux de NO (i.e. N₂/O₂) par l'appareil de délivrance de NO 1, en particulier le débit de NO, puisque les mesures de débit opérées par le capteur de débit 25 sont retournées, via la ligne de mesure de débit 26 (i.e. câbles électriques ou analogues) et le port de connexion au capteur 27, à des moyens de pilotage 210 à (micro)processeur de l'appareil de délivrance de NO 1, typiquement un contrôleur, qui traitent ces mesures de débit comme expliqué ci-après et illustré en Fig. 2. Le port de connexion au capteur 27 est relié électriquement aux moyens de pilotage 210 via une ou des liaisons électriques, par exemple des câbles électriques ou analogues.

L'appareil de fourniture de NO 1 comprend une carcasse rigide 1.1, par exemple en polymère, comprenant le circuit de gaz interne 200 sur Fig. 2, typiquement des lignes, passages ou conduits de gaz ou analogue, servant à acheminer le flux de gaz à base de NO, i.e. le mélange NO/N₂, provenant des bouteilles de mélange de NO/N₂ 12. Le circuit de gaz interne 200 relie fluidiquement l'entrée (ou les entrées) de gaz 21 de l'appareil de fourniture de NO 1 à la ligne d'injection 23 de manière à convoyer le flux de gaz à base de NO entre eux.

Dans le mode de réalisation schématisé en Fig. 2, une portion du circuit de gaz interne 200 comprend deux tronçons de gaz agencés en parallèle, à savoir un tronçon principal 200.1 et un tronçon secondaire 200.2, dit tronçon de secours. Le tronçon principal 200.1 et le tronçon secondaire 200.2 se raccordent fluidiquement l'un à l'autre et au reste du circuit de gaz 200 en des sites de raccordement amont 260 et aval 261 situés, respectivement, en amont et en aval de moyens de contrôle de débit principaux et secondaires 220, 221.

Dans ce cas, en mode de fonctionnement normal, le flux de NO/N₂ transite par le tronçon principal 200.1, alors qu'en cas d'interruption de transmission de signal, il passe automatiquement en mode secours, comme expliqué ci-après et :
- si les moyens de contrôle de débit principaux 220 restent opérationnels, c'est-à-dire continuent à fonctionner normalement, typiquement un contrôleur de débit massiqueou MFC, alors le flux de NO/N₂ continue à transiter par le tronçon principal 200.1,
- si les moyens de contrôle de débit principaux 220 sont rendus non-opérationnels, c'est-à-dire dysfonctionnels, le flux de NO/N₂ est dévié et transite alors par le tronçon de secours 200.2.

Bien entendu, selon un autre mode de réalisation (non montré), le circuit de gaz interne 200 pourrait être configuré différemment, par exemple comprendre une ligne de gaz unique en lieu et place des deux tronçons 200.1, 200.2, laquelle serait utilisée en mode de fonctionnement normal et en mode secours. Toutefois, dans ce mode de réalisation, un dysfonctionnement des moyens de contrôle de débit principaux 220 ne pourrait pas être pris en compte et l'appareil 1 deviendrait alors non-fonctionnel.

D'une façon générale, les moyens de contrôle de débit principaux et secondaires 220, 221, tels des moyens à vanne principaux et secondaires 2200, 2210, schématisés en Fig. 2, i.e. un (ou des) dispositif à vanne(s), par exemple une (ou des) électrovanne proportionnelle pilotée par les moyens de pilotage 210, sont agencés sur le circuit de gaz interne 200, notamment sur les tronçons principal 200.1 et secondaire 200.2, et servent à contrôler ou ajuster le flux gazeux qui y circule en direction de la ligne d'injection 23, c'est-à-dire vers le dispositif d'injection 24 et ce, que ce soit en mode de fonctionnement normal ou en mode de secours.

De préférence, le tronçon principal 200.1 comprend électrovanne proportionnelle 220 et un capteur de débit additionnel 230, typiquement un contrôleur de débit massique ou MFC, alors que le tronçon secondaire 200.2 comprend une (ou des) électrovanne de type tout ou rien (TOR) 221, de préférence pilotée en mode pulsée.

Préférentiellement, les moyens de contrôle de débit principaux et secondaires 220, 221 de l'appareil de fourniture de NO 1 sont commandés, i.e. contrôlés, par les moyens de pilotage 210, c'est-à-dire un (ou des) dispositif de pilotage ou (micro)contrôleur, agencés dans le boitier 1.1 de l'appareil de fourniture de NO 1.

Typiquement, les moyens de pilotage 210 comprennent une (des) carte électronique comprenant un (ou plusieurs) microprocesseur(s) 211 mettant en œuvre un ou des algorithmes. Les moyens de pilotage 210 permettent notamment d'ajuster ou contrôler le débit de gaz à base de NO en pilotant tout ou partie des moyens à vanne 2200, 2210, typiquement d'ouvrir ou fermer une ou des (électro)vannes, pour obtenir un débit de gaz à base de NO, typiquement autoriser ou stopper le débit de gaz.

Bien entendu, les moyens de pilotage 210 permettent en outre d'opérer des calculs et/ou de contrôler ou commander tous les éléments électromécaniques de l'appareil 1, tels que les capteurs, les affichages ...

Comme expliqué ci-après, les moyens de pilotage 210 peuvent déterminer le débit de NO à fournir pour obtenir la teneur en NO souhaitée dans le mélange combiné, c'est-à-dire la posologie en NO désirée, à partir notamment de la consigne de teneur en NO réglée et/ou fixée par l'utilisateur, de la composition du mélange gazeux NO/N₂, en particulier de la teneur en NO dans ce mélange gazeux NO/N₂, et d'une (ou des) mesure de débit opérée(s) par le capteur de débit 25 agencé sur la branche inspiratoire 21 et relié par une ligne de mesure de débit 26 au dispositif de fourniture de NO 1, en particulier aux moyens de pilotage 210, via le port de connexion au capteur 27.

Le circuit de gaz interne 200 de l'appareil de fourniture de NO 1 peut aussi comprendre d'autres éléments ou composants, en particulier un (ou des) capteur de pression 250, un (ou des) capteur de débit additionnel ou débitmètre et/ou des dispositifs à orifice calibré 240 ou autres. Ces autres éléments peuvent être agencés en amont et/ou en aval des moyens de contrôle de débit 220, 221, i.e. des moyens à vanne, par exemple on peut utiliser un capteur de débit additionnel pour déterminer le débit de gaz à base de NO circulant dans tout ou partie du circuit de gaz interne 200, notamment pour s'assurer qu'il est conforme au débit souhaité.

Ainsi, en Fig. 2, on voit que le tronçon principal 200.1 comprend un capteur de débit additionnel 230 agencé en amont des moyens de contrôle de débit 220, tels des moyens à vanne 2200, par exemple une (des) électrovanne, préférentiellement une électrovanne proportionnelle, contrôlant le passage de gaz dans le tronçon principal 200.1. Cet ensemble forme un contrôleur de débit massique (MFC).

Par ailleurs, le tronçon secondaire 200.2 comprend un dispositif à orifice calibré 240 agencé en aval de moyens de contrôle de débit secondaire 221, tels des moyens à vanne secondaire 2210, préférentiellement une (des) électrovanne, contrôlant le débit de passage de gaz dans le tronçon secondaire 200.2.

Avantageusement, l'électrovanne des moyens de contrôle de débit secondaire 221 est du type tout ou rien (TOR), c'est-à-dire pouvant adopter 2 positions « stables », à savoir une position ouverte laissant passer le flux gazeux et une position fermée empêchant toute circulation de flux gazeux.

Selon l'invention, lors d'une interruption de transmission de signal de mesure de débit de gaz respiratoire, c'est-à-dire de perte du signal de débit de gaz respiratoire, mais avec moyens de contrôle de débit principaux 220 opérationnels (MFC), c'est-à-dire qui continuent à fonctionner normalement, le flux de NO/N₂ continue à transiter par le tronçon principal 200.1 et les moyens de pilotage 210 commandent l'électrovanne proportionnelle 220, c'est-à-dire en mode proportionnel, pour ajuster et délivrer le gaz au débit de secours souhaité. L'électrovanne de type tout ou rien 221 (TOR) agencée sur le tronçon secondaire 200.2 est quant à elle alors en position fermée. L'appareil 1 est alors en mode secours mais le flux de NO/N₂ continue à transiter par le tronçon principal 200.1.

Par contre, en cas d'interruption de transmission de signal de mesure de débit de gaz respiratoire avec dysfonctionnement simultané des moyens de contrôle de débit principaux 220 (i.e. du MFC, par exemple de l'électrovanne proportionnelle), les moyens de pilotage 210 commandent (typiquement en mode pulsé) alors une ouverture de l'électrovanne de type tout ou rien 221 (TOR) agencée sur le tronçon secondaire 200.2 pour laisser passer le gaz dans ce tronçon secondaire 200.2 pour délivrer le gaz au débit de secours souhaité, alors que l'électrovanne proportionnelle 220 n'est plus commandée et passe en position de fermeture, qui est préférentiellement sa position par défaut. L'appareil 1 est alors aussi en mode secours et le flux de NO/N₂ ne transite plus par le tronçon principal 200.1.

Par ailleurs, le débitmètre ou capteur de débit additionnel 230 du MFC peut être du type à différentiel de pression, massique ou autre, et coopère avec les moyens de pilotage 210 pour leur fournir des mesures de débit du flux de NO/N₂.

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

L'alimentation électrique de l'appareil de fourniture de NO 1, en particulier des composants nécessitant du courant électrique pour fonctionner, tels les moyens de pilotage 210, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 à l'appareil de fourniture de NO 1. Elle vient se raccorder fluidiquement (en 61) à la ligne d'alimentation en gaz 21, entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue. La ligne de prélèvement de gaz 60 permet de prélever des échantillons de gaz et de les convoyer jusqu'au dispositif de fourniture de NO 1 où ils sont analysés dans un analyseur de gaz interne (non montré), c'est-à-dire au sein d'une ligne de calibration comprenant au moins un capteur, notamment une ou des cellules électrochimiques, relié électriquement aux moyens de pilotage, afin de vérifier leur conformité. En particulier, il convient de vérifier que la composition du gaz final est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, qu'il ne contient pas une teneur en NO₂ trop élevée et que sa teneur en NO correspond à la posologie souhaitée, i.e. dose de NO à administrer par inhalation qui est habituellement choisie par le personnel soignant, i.e. médecin ou analogue. Cette vérification de conformité se fait classiquement au moyen de moyens de mesure dédiés, typiquement des capteurs de NO₂, de NO et d'O₂, par exemple des cellules électrochimiques ou analogues, qui doivent eux-mêmes être calibrés périodiquement, par exemple toutes les semaines. Les moyens de pilotage 210 de l'appareil 1 sont en outre configurés pour récupérer et traiter, i.e. analyser, les signaux provenant des différents capteurs de l'analyseur de gaz, lequel est agencé dans l'appareil 1, et d'agir en réponse à ces signaux, notamment pour opérer une calibration des capteurs.

Dans le cadre de l'invention, afin de pouvoir déterminer un débit de NO de consigne, en l'absence de mesure de débit de gaz respiratoire, c'est-à-dire en cas d'interruption de la transmission aux moyens de pilotage 210 ou perte de signal, des mesures de débit de gaz respiratoire opérées par le capteur de débit 25, et dès lors de pouvoir fournir un mélange gazeux final à base de NO, i.e. mélange combiné, au patient contenant une proportion de NO égale ou proche d'une posologie fixée par le personnel soignant, i.e. un médecin ou analogue, on procède comme suit.

L'interruption de transmission des mesures de débit, c'est-à-dire la perte de signal, peut résulter d'un dysfonctionnement ou défaut du capteur de débit, d'une rupture de connectivité électrique comme un débranchement accidentel du capteur de débit ou un sectionnement d'un câble de liaison, ou d'une autre cause, comme des perturbations électromagnétiques ou autre.

Pendant le fonctionnement normal de l'appareil 1, c'est-à-dire avant toute interruption de transmission de mesure de débit provenant du capteur de débit 25, les moyens de pilotage 210 à (micro)processeur 211, tel un contrôleur, déterminent le débit de consigne de gaz contenant du NO à fournir au dispositif d'injection 24 et piloter des moyens de contrôle de débit 220, 221, telles des électrovannes proportionnelles 220 et/ou TOR 221, pour fournir le gaz contenant du NO au débit de consigne ayant été déterminé, comme expliqué ci-avant.

Cette détermination du débit de consigne de NO est réalisée à partir d'une mesure de débit de gaz respiratoire, i.e. valeur ou signal de débit, opérée et fournie par le capteur de débit 25 aux moyens de pilotage 210, d'une consigne de teneur en NO correspondant à la proportion finale de NO souhaitée dans le mélange gazeux combiné, typiquement fixée par l'utilisateur, i.e. personnel soignant, et de la proportion initiale de NO dans le gaz contenant du NO alimentant l'appareil de délivrance de NO 1, c'est-à-dire la quantité de NO présente dans le mélange NO/N₂ provenant des bouteilles de gaz 10, typiquement comprise entre 200 et 1000 ppmv, par exemple 450 ou 800 ppmv.

La valeur de consigne de NO et/ou la teneur en NO dans le mélange gazeux NO/N₂ alimentant l'appareil 1 peuvent être entrées et/ou ajustées et/ou modifiées par l'utilisateur, par exemple via l'IHM, grâce à des moyens de réglage de dose ou analogue, telles des touches, des curseurs ou autres. Préférentiellement, la valeur de consigne de NO et/ou la teneur en NO dans le mélange gazeux NO/N₂ alimentant l'appareil 1 peuvent être mémorisées par les moyens de mémorisation 212 de l'appareil 1.

Avantageusement, le débit de consigne de NO est déterminé, i.e. réactualisé, à une fréquence comprise entre 10 Hz et 1000 Hz ou à période temporelle comprise entre 1 et 100 millisecondes (msec).

Les débits de consigne de NO successifs ainsi déterminés pendant le fonctionnement normal de l'appareil 1, sont mémorisés par des moyens de mémorisation 212, i.e. un dispositif de mémorisation, telle une mémoire flash ou autre. Ces débits de consigne de NO servent à piloter les moyens de contrôle de débit 220, 221 agencés sur le circuit interne 200, en particulier sur le tronçon principal 200.1 et le tronçon secondaire 200.2, i.e. le tronçon de secours, comme expliqué ci-dessus, pour fournir le gaz contenant du NO au débit de consigne ayant été déterminé.

Comme déjà dit, en mode de fonctionnement normal, dans le mode de réalisation de Fig. 2, le flux de NO/N₂ transite par le tronçon principal 200.1 puisque les moyens de contrôle de débit principaux 220, typiquement des moyens à vannes principaux 2200, du tronçon principal 200.1 sont en position ouverte pour laisser passer le flux gazeux, alors que les moyens de contrôle de débit secondaires 221, i.e. les moyens à vannes secondaires 2200, du tronçon secondaire 200.2 sont en position fermée pour empêcher toute circulation de gaz dans le tronçon secondaire 200.2, c'est-à-dire bloquer ou interdire toute circulation de flux gazeux à base de NO au sein de ce tronçon secondaire 200.2.

Selon l'invention, en cas d'interruption de transmission aux moyens de pilotage 210, de toute mesure de débit de gaz respiratoire par le capteur de débit 25, l'appareil de délivrance de NO 1 passe en mode secours et les moyens de pilotage 210 sont alors configurés pour piloter, contrôler ou commander les moyens de contrôle de débit principaux et secondaires 220, 221, typiquement les moyens à vannes principaux et secondaires 2210, 2200, pour fournir le gaz contenant du NO à un débit de secours obtenu ou calculé à partir d'un ou plusieurs débits de consigne de gaz contenant du NO ayant été déterminés par les moyens de pilotage 210 et ayant été mémorisés par les moyens de mémorisation 212, avant ladite interruption de transmission.

Dans le mode de réalisation de Fig. 2, en cas d'interruption de transmission aux moyens de pilotage 210, de toute mesure de débit de gaz respiratoire par le capteur de débit 25, le flux de NO/N₂ transite alors par le tronçon principal 200.1 ou par le secondaire 200.2 selon que les moyens de contrôle de débit principaux 220, i.e. les moyens à vannes principaux 2200, du tronçon principal 200.1 dysfonctionnent aussi ou non, comme déjà expliqué.

Ainsi, lorsque les moyens de contrôle de débit principaux 220 du tronçon principal 200.1, telle une électrovanne proportionnelle d'un MFC, fonctionnent normalement les moyens de pilotage 210 sont configurés pour contrôler les moyens de contrôle de débit principaux et/ou secondaires 220, 221 pour faire circuler le flux gazeux aux travers desdits moyens de contrôle de débit principaux 220 et délivrer le débit gaz de secours via le tronçon principal 200.1.

Par contre, en cas de dysfonctionnement aussi des moyens de contrôle de débit principaux 220 du tronçon principal 200.1, telle une électrovanne proportionnelle, lesdits moyens de contrôle de débit principaux 220, i.e. les moyens à vannes principaux 2200, du tronçon principal 200.1 passent (par défaut) en position fermée pour stopper le flux gazeux, alors que les moyens de contrôle de débit secondaires 221, i.e. les moyens à vannes secondaires 2100, du tronçon secondaire 200.2, telle une électrovanne TOR, sont commandés par les moyens de pilotage 210 pour être en position ouverte et permettre ainsi une circulation de gaz dans le tronçon secondaire 200.2 et sa délivrance débit gaz de secours, après passage dans le dispositif à orifice calibré 240.

Autrement dit, l'appareil de délivrance de NO 1 est donc conçu pour fonctionner selon (au moins) deux modes de fonctionnement, à savoir un mode de fonctionnement dit « normal » et un mode de fonctionnement dit « de secours », appelé « mode secours ». Le passage du mode de fonctionnement normal au mode de secours se fait automatiquement en réponse à une détection par les moyens de pilotage 210 d'une interruption de transmission des mesures de débit de gaz respiratoire opérées par le capteur de débit 25.

Dans tous les cas, en cas d'interruption de transmission de signal, le débit de secours est obtenu ou calculé à partir de plusieurs débits de consigne ayant été déterminés et mémorisés préalablement, pendant une durée dt donnée, par les moyens de pilotage 210, pendant le fonctionnement normal de l'appareil 1, c'est-à-dire avant ladite interruption de transmission et passage en mode secours.

La durée dt correspond à la période de temps précédant le moment de l'interruption de transmission du signal de débit, c'est-à-dire la détection par les moyens de pilotage 210 de l'interruption de transmission des mesures de débit. De préférence, la durée dt est inférieure ou égale à 30 secondes, de préférence inférieure ou égale à 20 secondes, par exemple inférieure ou égale à 10 secondes.

En d'autres termes, on n'utilise pas toutes les valeurs de débits de consigne ayant été déterminées et mémorisées pendant le fonctionnement normal de l'appareil 1 qui dure généralement plusieurs heures, mais uniquement les plus récentes, à savoir celles déterminées et mémorisées pendant la durée dt, c'est-à-dire la période de temps de durée courte, typiquement < 30 sec, qui précède immédiatement le moment ou instant où l'interruption de transmission des mesures (i.e. signal ou valeur) de débit s'est produite et a été détectée par les moyens de pilotage.

Les valeurs de débits de consigne ayant été déterminées et mémorisées pendant cette durée dt, sont de préférence moyennées, et éventuellement pondérées ou autres, pour obtenir une valeur moyenne de débit, i.e. le débit de secours.

On comprend donc que, selon l'invention, la (les) valeur de débit de secours est calculée par partir des valeurs de débit de consigne de NO ayant été précédemment utilisées pour contrôler le flux de NO, i.e. débit, pendant le fonctionnement normal de l'appareil 1, et mémorisée par les moyens de mémorisation de l'appareil 1, pendant durée dt, et ce, contrairement à ce qui est enseigné par l'art antérieur, qui préconise d'utiliser, en cas d'interruption de transmission du signal de débit, soit une unique valeur de débit de secours constante préfixée, soit des valeurs de débit du flux de gaz respiratoire (e.g. air ou N₂/O₂) ou du flux de NO mesurées pendant le fonctionnement normal de l'appareil 1 et enregistrées dans un historique mémorisé.

Autrement dit, il n'a jamais été préconisé avant la présente invention, de mémoriser et utiliser des valeurs de débit de consigne de NO ayant été précédemment utilisées pour contrôler le flux de NO, pendant le fonctionnement normal de l'appareil 1, afin de les utiliser ensuite en cas d'interruption de la transmission des mesures de débit (i.e. perte du signal de débit) provenant du capteur de débit de flux respiratoire, e.g. air ou O₂/N₂, c'est-à-dire après passage en mode secours.

Selon l'invention, le débit de secours correspond préférentiellement à une valeur moyenne de débit obtenue à partir des valeurs de débits de consigne déterminées et mémorisées, pendant la durée dt, en général une durée dt de moins 45 à 60 secondes, typiquement moins de 30 secondes, par exemple pendant 10 secondes ou une autre durée, lorsque l'appareil 1 était pleinement fonctionnel, comme expliqué ci-avant, c'est-à-dire lorsqu'il est en mode de fonctionnement normal.

Une fois le débit de secours déterminé, les moyens de pilotage 210 pilotent les moyens de contrôle de débit principaux et secondaires 220, 221, typiquement les moyens à vannes principaux et secondaires 2200, 2210, pour fournir le gaz contenant du NO, c'est-à-dire le mélange gazeux NO/N₂, au débit de secours ainsi obtenu, comme expliqué ci-avant.

L'appareil de délivrance de NO 1 peut alors continuer à fournir le mélange à base de NO, i.e. mélange NO/N₂, mais au débit de secours ainsi déterminé et ce, malgré l'absence de mesures de débit de gaz respiratoire, c'est-à-dire malgré l'interruption de transmission de ces mesures de débit (perte de signal), voire même en cas de défaillance des moyens de contrôle de débit principaux 220, tel le MFC, ce qui permet de continuer à soigner le patient en lui fournissant le NO à la posologie désirée ou proche de cette posologie. Ceci améliore grandement la sécurité de traitement pour le patient.

Une installation d'administration de gaz 100 peut être utilisée pour administrer par inhalation du monoxyde d'azote (NO), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

1. Installation (100) de fourniture d'un mélange gazeux contenant du NO à un patient comprenant :
- un appareil de délivrance de NO (1) alimenté en un gaz contenant du NO en une proportion initiale donnée, et configuré pour fournir le gaz contenant du NO,
- un circuit respiratoire (20 ; 21) comprenant un dispositif d'injection (24) configuré pour opérer un mélange du gaz contenant du NO provenant de l'appareil de délivrance de NO (1) avec un flux de gaz respiratoire contenant de l'O₂ acheminé par le circuit respiratoire (20 ; 21), et obtenir un mélange gazeux combiné contenant du NO et de l'oxygène, et
- un capteur de débit (25) configuré pour mesurer au moins un débit de gaz respiratoire au sein du circuit respiratoire (20 ; 21) et fournir au moins une mesure de débit de gaz respiratoire,
et dans laquelle l'appareil de délivrance de NO (1) comprend :
- un circuit de gaz interne (200) pour acheminer le gaz contenant du NO,
- des moyens de contrôle de débit principaux et secondaires (220, 221) configurés pour contrôler le débit de gaz contenant du NO acheminé par le circuit de gaz interne (200),
- des moyens de pilotage (210) à processeur (211) configurés pour :
▪ déterminer au moins un débit de consigne de gaz contenant du NO à fournir au dispositif d'injection (24), à partir d'au moins une mesure de débit de gaz respiratoire opérée et fournie par le capteur de débit (25), et
▪ piloter au moins une partie des moyens de contrôle de débit (220, 221) pour fournir le gaz contenant du NO au débit de consigne ayant été déterminé,
- et des moyens de mémorisation (212),
**caractérisée en ce que** :
- les moyens de mémorisation (212) sont configurés pour mémoriser des débits de consigne successifs de gaz contenant du NO ayant été déterminés par les moyens de pilotage (210), et
- en cas d'interruption de transmission aux moyens de pilotage (210), de toute mesure de débit de gaz respiratoire par le capteur de débit (25), les moyens de pilotage (210) sont configurés pour piloter lesdits moyens de contrôle de débit (220, 221) pour fournir le gaz contenant du NO à un débit de secours obtenu ou calculé à partir d'un ou plusieurs débits de consigne de gaz contenant du NO ayant été déterminés par les moyens de pilotage (210) et mémorisés par les moyens de mémorisation (212), avant ladite interruption de transmission.

2. Installation selon la revendication 1, **caractérisée en ce que** les moyens de pilotage (210) sont configurés pour déterminer des débits de consigne de gaz contenant du NO successifs à partir de plusieurs mesures de débit de gaz respiratoire successives opérées par le capteur de débit (25) et fournies auxdits moyens de pilotage (210).

3. Installation selon la revendication 2, **caractérisée en ce que** les moyens de mémorisation (212) sont configurés pour mémoriser les débits de consigne de gaz contenant du NO successifs ayant été déterminés par les moyens de pilotage (210).

4. Installation selon la revendication 1, **caractérisée en ce que** le débit de secours est obtenu ou calculé à partir de plusieurs débits de consigne ayant été déterminés par les moyens de pilotage (210) pendant une durée (dt) donnée, avant ladite interruption de transmission.

5. Installation selon la revendication 4, **caractérisée en ce que** le débit de secours est calculé en opérant une moyenne des débits de consigne ayant été mémorisés pendant la durée donnée (dt).

6. Installation selon la revendication 4, **caractérisée en ce que** la durée (dt) donnée est inférieure ou égale à 30 secondes, de préférence inférieure ou égale à 20 secondes, de préférence encore inférieure ou égale à 10 secondes.

7. Installation selon la revendication 1, **caractérisée en ce que** les moyens de pilotage (210) sont configurés pour déterminer ledit au moins un débit de consigne de gaz contenant du NO à fournir au dispositif d'injection (24), à partir d'au moins une mesure de débit de gaz respiratoire opérée et fournie par le capteur de débit (25), d'une consigne de teneur en NO correspondant à la proportion finale de NO souhaitée dans le mélange gazeux combiné, et de la proportion initiale de NO dans le gaz contenant du NO alimentant l'appareil de délivrance de NO (1).

8. Installation selon la revendication 1, **caractérisée en ce que** l'appareil de délivrance de NO (1) est alimenté en un gaz contenant une proportion initiale de NO comprise entre 100 et 1500 ppmv, en particulier un mélange gazeux formé d'azote et de NO.

9. Installation selon la revendication 1, **caractérisée en ce que** l'appareil de délivrance de NO (1) comprend des moyens de réglage de dose configurés pour permettre à un utilisateur de fixer ou sélectionner la consigne de teneur en NO correspondant à la proportion finale de NO souhaitée dans le mélange gazeux combiné.

10. Installation selon la revendication 1, **caractérisée en ce qu'**elle comprend un ventilateur médical (50) configuré pour fournir le flux de gaz respiratoire contenant de l'O₂ audit circuit respiratoire (20 ; 21).

11. Installation selon l'une des revendications 4 à 6, **caractérisée en ce que** la durée (dt) donnée est fixe et mémorisée par les moyens de mémorisation (212), de préférence les moyens de mémorisation sont intégrés aux moyens de pilotage (210).

12. Installation selon la revendication 9, **caractérisée en ce que** la consigne de teneur en NO est comprise entre 1 et 80 ppmv, typiquement entre 5 et 40 ppmv.

13. Installation selon la revendication 9, **caractérisée en ce que** les moyens de réglage de dose font partie d'une interface homme-machine (IHM) ou d'une interface graphique utilisateur (IGU).

14. Installation selon la revendication 9, **caractérisée en ce que** les moyens de réglage de dose comprennent une ou des touches tactiles, actionnables par l'utilisateur, s'affichant sur un écran digital à dalle tactile.

15. Installation selon la revendication 1, **caractérisée en ce que** les moyens de pilotage (210) comprennent au moins un microprocesseur agencé sur au moins une carte électronique.

## Patentansprüche

1. Anlage (100) zur Abgabe eines NO-haltigen Gasgemisches an einen Patienten, umfassend:
- eine NO-Abgabevorrichtung (1), die mit einem NO-haltigen Gas in einem vorgegebenen Ausgangsanteil versorgt wird und dazu ausgelegt ist, das NO-haltige Gas abzugeben,
- einen Atemkreislauf (20; 21), der eine Injektionsvorrichtung (24) umfasst, die so ausgelegt ist, dass sie das NO-haltige Gas aus der NO-Abgabevorrichtung (1) mit einem O₂ ,das durch den Atemkreislauf (20; 21) geleitet wird, und zur Erzeugung eines kombinierten Gasgemisches, das NO und Sauerstoff enthält, sowie
- einen Durchflusssensor (25), der so ausgelegt ist, dass er mindestens einen Atemgasdurchfluss innerhalb des Atemkreises (20; 21) misst und mindestens einen Atemgasdurchflusswert liefert,
und wobei die NO-Abgabevorrichtung (1) umfasst:
- einen internen Gaskreislauf (200) zum Leiten des NO-haltigen Gases,
- primäre und sekundäre Durchflusssteuerungsmittel (220, 221), die so konfiguriert sind, dass sie den Durchfluss des durch den internen Gaskreislauf (200) geleiteten NO-haltigen Gases steuern,
- eine Steuerungseinrichtung (210) mit einem Prozessor (211), die so konfiguriert ist, dass sie:
▪ die Bestimmung mindestens eines Sollwerts für den Durchfluss des NO-haltigen Gases, das der Injektionsvorrichtung (24) zugeführt werden soll, ausgehend von mindestens einer vom Durchflusssensor (25) durchgeführten und gelieferten Messung des Atemgasdurchflusses, und
▪ zum Ansteuern mindestens eines Teils der Durchflusssteuerungsmittel (220, 221), um das NO-haltige Gas mit dem ermittelten Soll-Durchfluss bereitzustellen,
- und Speichermittel (212),
**dadurch gekennzeichnet, dass**:
- die Speichermittel (212) so konfiguriert sind, dass sie aufeinanderfolgende Soll-Durchflussmengen des NO-haltigen Gases speichern, die von den Steuermitteln (210) bestimmt wurden, und
- im Falle einer Unterbrechung der Übertragung von Atemgasdurchflussmesswerten durch den Durchflusssensor (25) an die Steuermittel (210), die Steuermittel (210) so konfiguriert sind, dass sie die Durchflusssteuermittel (220, 221) so steuern, dass das NO-haltige Gas mit einem Notdurchfluss bereitgestellt wird, der aus einem oder mehreren Soll-Durchflusswerten für NO-haltiges Gas ermittelt oder berechnet wurde, die von den Steuermitteln (210) bestimmt und von den Speichermitteln (212) gespeichert wurden, vor der genannten Übertragungsunterbrechung.

2. Anlage nach Anspruch 1, **dadurch gekennzeichnet, dass** die Steuermittel (210) so konfiguriert sind, dass sie aufeinanderfolgende Soll-Durchflussraten für NO-haltiges Gas aus mehreren aufeinanderfolgenden Messungen des Atemgasdurchflusses bestimmen, die vom Durchflusssensor (25) durchgeführt und den Steuermitteln (210) zugeführt werden.

3. Anlage nach Anspruch 2, **dadurch gekennzeichnet, dass** die Speichermittel (212) so konfiguriert sind, dass sie die aufeinanderfolgenden Soll-Durchflussraten für NO-haltiges Gas speichern, die von den Steuermitteln (210) bestimmt wurden.

4. Anlage nach Anspruch 1, **dadurch gekennzeichnet, dass** der Notdurchfluss aus mehreren Soll-Durchflüssen ermittelt oder berechnet wird, die von den Steuermitteln (210) während einer vorgegebenen Zeitdauer (dt) vor der genannten Übertragungsunterbrechung bestimmt wurden.

5. Anlage nach Anspruch 4, **dadurch gekennzeichnet, dass** der Ersatzdurchfluss durch Mittelwertbildung der während der vorgegebenen Zeitdauer (dt) gespeicherten Soll-Durchflussmengen berechnet wird.

6. Anlage nach Anspruch 4, **dadurch gekennzeichnet, dass** die vorgegebene Zeitdauer (dt) kleiner oder gleich 30 Sekunden, vorzugsweise kleiner oder gleich 20 Sekunden, noch bevorzugter kleiner oder gleich 10 Sekunden ist.

7. Anlage nach Anspruch 1, **dadurch gekennzeichnet, dass** die Steuermittel (210) so konfiguriert sind, dass sie den mindestens einen Sollwert für den Durchfluss von NO-haltigem Gas, der der Einspritzvorrichtung (24) zuzuführen ist, ausgehend von mindestens einer vom Durchflusssensor (25) durchgeführten und gelieferten Messung des Atemgasdurchflusses, einem Sollwert für den NO-Gehalt, der dem gewünschten Endanteil an NO im kombinierten Gasgemisch entspricht, und dem Anfangsanteil an NO in dem NO-haltigen Gas, das der NO-Abgabevorrichtung (1) zugeführt wird.

8. Anlage nach Anspruch 1, **dadurch gekennzeichnet, dass** die NO-Abgabevorrichtung (1) mit einem Gas versorgt wird, das einen anfänglichen NO-Anteil zwischen 100 und 1500 ppmv enthält, insbesondere mit einem Gasgemisch aus Stickstoff und NO.

9. Anlage nach Anspruch 1, **dadurch gekennzeichnet, dass** die NO-Abgabevorrichtung (1) Dosierungsregelungsmittel umfasst, die so konfiguriert sind, dass ein Benutzer den Sollwert für den NO-Gehalt festlegen oder auswählen kann, der dem gewünschten Endanteil an NO im kombinierten Gasgemisch entspricht.

10. Anlage nach Anspruch 1, **dadurch gekennzeichnet, dass** sie einen medizinischen Ventilator (50) umfasst, der so ausgelegt ist, dass er den O□-haltigen Atemgasstrom an den Atemkreislauf (20; 21) liefert.

11. Anlage nach einem der Ansprüche 4 bis 6, **dadurch gekennzeichnet, dass** die vorgegebene Dauer (dt) fest ist und von den Speichermitteln (212) gespeichert wird, wobei die Speichermittel vorzugsweise in die Steuermittel (210) integriert sind.

12. Anlage nach Anspruch 9, **dadurch gekennzeichnet, dass** der Sollwert für den NO-Gehalt zwischen 1 und 80 ppmv, typischerweise zwischen 5 und 40 ppmv, liegt.

13. Anlage nach Anspruch 9, **dadurch gekennzeichnet, dass** die Dosierungsregelungsmittel Teil einer Mensch-Maschine-Schnittstelle (MMS) oder einer grafischen Benutzeroberfläche (GUI) sind.

14. Anlage nach Anspruch 9, **dadurch gekennzeichnet, dass** die Mittel zur Dosiseinstellung eine oder mehrere vom Benutzer betätigbare Touch-Tasten umfassen, die auf einem digitalen Touchscreen-Display angezeigt werden.

15. Anlage nach Anspruch 1, **dadurch gekennzeichnet, dass** die Steuermittel (210) mindestens einen Mikroprozessor umfassen, der auf mindestens einer elektronischen Platine angeordnet ist.

## Claims

1. An installation (100) for delivering a gas mixture containing NO to a patient, comprising:
- an NO delivery device (1) supplied with a gas containing NO in a given initial proportion, and configured to deliver the NO-containing gas,
- a breathing circuit (20; 21) comprising an injection device (24) configured to mix the NO-containing gas from the NO delivery device (1) with a flow of O□-containing breathing gas conveyed by the breathing circuit (20; 21), and to obtain a combined gas mixture containing NO and oxygen, and
- a flow sensor (25) configured to measure at least one respiratory gas flow rate within the respiratory circuit (20; 21) and provide at least one respiratory gas flow rate measurement,
and wherein the NO delivery device (1) comprises:
- an internal gas circuit (200) for conveying the NO-containing gas,
- primary and secondary flow control means (220, 221) configured to control the flow of NO-containing gas delivered by the internal gas circuit (200),
- control means (210) with a processor (211) configured to:
▪ determine at least one setpoint flow rate of NO-containing gas to be supplied to the injection device (24), based on at least one measurement of respiratory gas flow performed and provided by the flow sensor (25), and
▪ control at least a portion of the flow control means (220, 221) to supply the NO-containing gas at the determined setpoint flow rate,
- and storage means (212),
**characterized in that**:
- the storage means (212) are configured to store successive setpoint flow rates of NO-containing gas determined by the control means (210), and
- in the event of an interruption in the transmission to the control means (210) of any measurement of respiratory gas flow by the flow sensor (25), the control means (210) are configured to control said flow control means (220, 221) to supply the NO-containing gas at a backup flow rate obtained or calculated from one or more setpoint flow rates of NO-containing gas that have been determined by the control means (210) and stored by the storage means (212), prior to said transmission interruption.

2. Installation according to claim 1, **characterized in that** the control means (210) are configured to determine successive setpoint flow rates of NO-containing gas based on a plurality of successive respiratory gas flow measurements taken by the flow sensor (25) and supplied to said control means (210).

3. Installation according to claim 2, **characterized in that** the storage means (212) are configured to store the successive setpoint flow rates of NO-containing gas that have been determined by the control means (210).

4. Installation according to claim 1, **characterized in that** the backup flow rate is obtained or calculated based on a plurality of setpoint flow rates determined by the control means (210) over a given duration (dt) prior to said transmission interruption.

5. Installation according to claim 4, **characterized in that** the backup flow rate is calculated by averaging the setpoint flow rates that have been stored during the given time period (dt).

6. Installation according to claim 4, **characterized in that** the given time period (dt) is less than or equal to 30 seconds, preferably less than or equal to 20 seconds, and more preferably less than or equal to 10 seconds.

7. Installation according to claim 1, **characterized in that** the control means (210) are configured to determine said at least one setpoint flow rate of NO-containing gas to be supplied to the injection device (24), based on at least one respiratory gas flow measurement taken and provided by the flow sensor (25), a setpoint NO concentration corresponding to the desired final proportion of NO in the combined gas mixture, and the initial proportion of NO in the NO-containing gas supplying the NO delivery device (1).

8. Installation according to claim 1, **characterized in that** the NO delivery device (1) is supplied with a gas containing an initial proportion of NO between 100 and 1500 ppmv, in particular a gas mixture consisting of nitrogen and NO.

9. Installation according to claim 1, **characterized in that** the NO delivery device (1) comprises dose adjustment means configured to allow a user to set or select the NO content setpoint corresponding to the desired final proportion of NO in the combined gas mixture.

10. Installation according to claim 1, **characterized in that** it comprises a medical ventilator (50) configured to supply the O□-containing respiratory gas flow to said respiratory circuit (20; 21).

11. Installation according to any one of claims 4 to 6, **characterized in that** the given duration (dt) is fixed and stored by the storage means (212), preferably the storage means are integrated into the control means (210).

12. Installation according to claim 9, **characterized in that** the target NO concentration is between 1 and 80 ppmv, typically between 5 and 40 ppmv.

13. Installation according to claim 9, **characterized in that** the dose control means are part of a human-machine interface (HMI) or a graphical user interface (GUI).

14. Installation according to claim 9, **characterized in that** the dose adjustment means comprise one or more touch-sensitive keys, operable by the user, displayed on a digital touchscreen.

15. Installation according to claim 1, **characterized in that** the control means (210) comprise at least one microprocessor arranged on at least one electronic board.
